# EUROPÄISCHE PATENTSCHRIFT

(11) **EP 0 868 425 B1**
(45) Veröffentlichungstag und Bekanntmachung des Hinweises auf die Patenterteilung: **06.10.2004**
(21) Anmeldenummer: 96943972.8
(22) Anmeldetag: 16.12.1996
(51) Int. Cl.: C07F 9/40, A61K 31/66

(54) **PHOSPHOLIPID-DERIVATE VON PHOSPHONOCARBONSÄUREN, DEREN HERSTELLUNG SOWIE DEREN VERWENDUNG ALS ANTIVIRALE ARZNEIMITTEL**
PHOSPHOLIPID DERIVATIVES OF PHOSPHONO-CARBOXYLIC ACIDS, THE PRODUCTION OF SAID DERIVATIVES AND THE USE OF SAID DERIVATIVES AS ANTIVIRAL MEDICAMENTS
DERIVES PHOSPHOLIPIDIQUES D'ACIDES PHOSPHONOCARBOXYLIQUES, LEUR PRODUCTION ET LEUR UTILISATION COMME MEDICAMENTS ANTIVIRAUX

(30) Priorität: 15.12.1995 DE 19547023; 22.10.1996 DE 19643416
(43) Veröffentlichungstag der Anmeldung: 07.10.1998
(73) Patentinhaber: Heidelberg Pharma Holding GmbH, 69126 Heidelberg (DE)
(72) Erfinder: ZILCH, Harald, D-68305 Mannheim (DE); HERRMANN, Dieter, D-69126 Heidelberg (DE); OPITZ, Hans-Georg, D-69469 Weinheim (DE); ZIMMERMANN, Gerd, D-68309 Mannheim (DE)
(74) Vertreter: Patentanwälte Zellentin & Partner
(86) Internationale Anmeldenummer: PCT/EP1996/005647
(87) Internationale Veröffentlichungsnummer: WO 1997/022613

(56) Entgegenhaltungen:
- EP-A- 0 050 327
- EP-A- 0 416 401
- WO-A-92/03462
- WO-A-96/15132

## Beschreibung

Gegenstand der vorliegenden Erfindung sind neue Lipid-Derivate von Phosphonocarbonsäuren und deren Ester der allgemeinen Formel I, 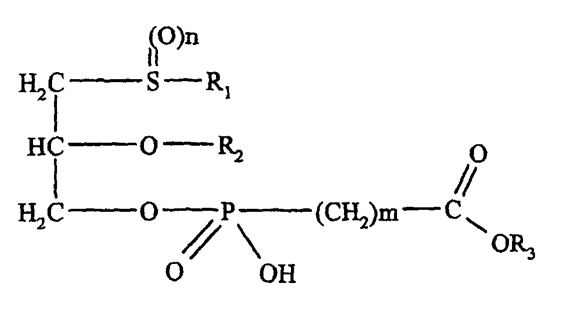 in der
- R¹: eine geradkettige oder verzweigte, gesättigte oder ungesättigte Alkylkette mit 9-13 Kohlenstoffatomen,
- R²: eine geradkettige oder verzweigte, gesättigte oder ungesättigte Alkylkette mit 8-12 Kohlenstoffatomen sein kann,
- R³: H, eine geradkettige oder verzweigte Alkykette mit 1-6 Kohlenstoffatomen, im besondern Methyl, Ethyl, Propyl, Isopropyl, Butyl, Isobutyl, t-Butyl, Pentyl, Hexyl, Neopentyl, Thexyl oder Phenyl, Cholin, Ethanolamin, Carnitin, C₅-C₇-Cycloalkylrest, Benzyl oder eine der folgenden Gruppen 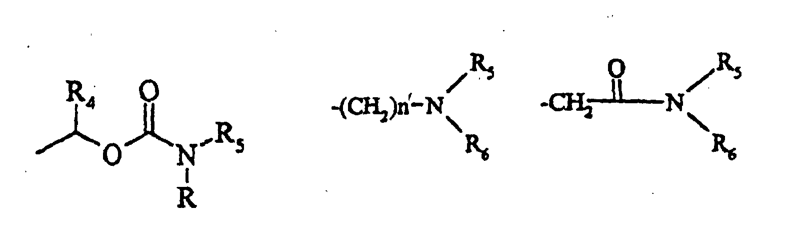 wobei R₄ Alkyl, Benzyl oder Phenyl und R₅ und R₆ Alkyl und n' 1,2 oder 3 bedeuten kann,
- n: 0, 1 oder 2 bedeutet und
- m: 0 bzw. für 1 bis 3 steht,
deren Tautomere, deren physiologisch verträgliche Salze anorganischer und organischer Basen, sowie Verfahren zu ihrer Herstellung und diese Verbindungen enthaltende Arzneimittel.

Da die Verbindungen der allgemeinen Formel I asymmetrische Kohlenstoffatome enthalten, sind auch sämtliche optisch aktiven Formen und racemische Gemische dieser Verbindungen Gegenstand der vorliegenden Erfindung.

Unter Verbindungen der Formel I werden im folgenden auch Salze, Tautomere, Ester, optisch aktive Formen und racemische Gemische verstanden.

Die Therapie bösartiger Neoplasien (Karzinome, Sarkome, hämatologische Neoplasien), entzündlicher Erkrankungen oder Autoimmunerkrankungen sowie von durch Viren oder Retroviren hervorgerufenen Erkrankungen, wie beispielsweise von AIDS, ARC (AIDS related complex), Cytomegalie-, Herpes-Infektionen oder Hepatitis, ist neben der unzureichenden Wirksamkeit der eingesetzten therapeutischen Wirkstoffe häufig auch mit deren extremen Nebenwirkungen verbunden. Dieser Effekt ist mit der zu geringen In-vivo-Selektivität bzw. der eingeschränkten therapeutischen Breite der eingesetzten pharmakologisch aktiven Substanzen zu erklären. Die günstigen pharmakologischen In-vitro-Eigenschaften der pharmakologisch aktiven Substanzen sind oft nicht auf die In-vivo-Verhältnisse übertragbar.

Seit Jahren versucht man deshalb durch Modifizierung der chemischen Struktur von pharmakologisch aktiven Substanzen neue Substanzen zur Verfügung zu stellen, die verbesserte Eigenschaften hinsichtlich der therapeutischen Breite aufweisen. Ferner werden oft neue pharmazeutische Darreichungsformen mit dem Ziel entwickelt, die aktiven Substanzen gezielt an ihren Wirkungsort zu transportieren, an dem sie ihre therapeutische Wirkung entfalten sollen. Dabei soll insbesondere die unerwünschte Wechselwirkung mit gesunden Zellen vermieden werden. Eine Möglichkeit zur Verbesserung der therapeutischen Breite besteht darin, durch geringfügige Modifizierung der pharmakologisch aktiven Substanz, beispielsweise durch Herstellung von Säure- oder Basenadditionssalzen oder durch Herstellung von pharmakologisch vertretbaren Estern [beispielsweise Fettsäureester; J. Pharm. Sci. 79, 531 (1990)], die physikalischen Eigenschaften der zugrundeliegenden aktiven Substanz derart zu verändern, daß die Löslichkeit oder Verträglichkeit der aktiven Substanz verbessert wird. Diese geringfügig chemisch modifizierten Verbindungen werden oft auch als "Prodrugs" bezeichnet, da sie beim Kontakt mit Körperflüssigkeiten oder in der Leber (first pass-Metabolismus) nahezu unmittelbar in das therapeutisch aktive Agens umgewandelt werden. Solche "Prodrugs" der Verbindungen der allgemeinen Formel I werden von der Erfindung mitumfaßt

Zur Verbesserung der katabolischen Stabilität wurden Nucleoside, wie z.B. ara-C und ara-A chemisch an Phospholipide gebunden. Die entsprechenden Derivate zeigten geringere Toxizität und höhere Stabilität in vivo im Vergleich zu den unmodifizierten Nucleosiden Absorption und Zellpenetration zeigten sich aber kaum beeinflußt [J. Med. Chem. 32, 367 (1989), Cancer Res. 37, 1640 (1977) und 41, 2707 (1981)]. Weitere Phospholipid-Derivate von Nucleosiden kennt man beispielsweise aus folgenden Literaturstellen:

In J. Biol. Chem. 265, 6112 (1990) ist die Herstellung und Verwendung von Liponucleotiden als antivirale Arzneimittel beschrieben. Untersucht und synthetisiert wurden hier aber nur die an bekannte Nucleoside, wie z.B. AZT und ddC, gekoppelten Dimyristoylphosphatidyl- und Dipalmitoylphosphatidylreste mit ihrer Fettsäureesterstruktur.

In J. Med. Chem. 33, 1380 (1990) sind Nucleosid-Konjugate von Thioetherlipiden mit Cytidindiphosphat beschrieben, die eine antitumorale Wirkung aufweisen und Verwendung in der Onkologie finden könnten.

In Chem. Pharm. Bull. 36, 209 (1988) sind 5'-(3-SN-Phosphatidyl)-nucleoside mit antileukämischer Aktivität beschrieben sowie deren enzymatische Synthese aus den entsprechenden Nucleosiden und Phosphocholinen in Gegenwart von Phospholipase D mit Transferaseaktivität.

Die enzymatische Synthese von Liponucleotiden ist u.a. ebenfalls in Tetrahedron Lett 28, 199 (1987) und Chem. Pharm. Bull. 36, 5020 (1988) beschrieben.

In WO 94/13324 sind oral verfügbare Wirkstoffe mit 1-O-Alkyl-, 1-O-Acyl-, 1-S-Acyl- und 1-S-Alkyl-sn-glycero-3-phosphaten als Lipid-Carrier beschrieben

Die Anmeldung EP 418814 sowie J Med. Chem. 34, 1912 (1991) beschreiben Isoprenoidphosphinylhydroxyformiate als Squalen-Synthetase-Inhibitoren.

In Biochem. Biophys. Res. Commun. 171, 458 (1990) ist mit Palmitylphosphonoformiat ein Lipid-Konjugat des antiretroviralen Foscarnets beschrieben und in J. Med. Chem. 20, 660 (1977) wird die Anti-HIV-Aktivität von (Hexyloxy)-hydroxyphosphinylhydroxyessigsäure aufgezeigt.

Es ist allgemein sehr hilfreich, effektive Wege zu finden therapeutische Arzneimittel-Konzentrationen in die entsprechenden Zielorgane bzw. Zielzellen zu transportieren, bei AIDS z.B. in die Zellen des Immunsystems und des lymphatischen Systems, die als Hauptreservoir der Virusreplikation gelten.

PFA (Phosphonoformic acid) und PAA (Phosphonoacetic acid) zeigen gute antivirale Aktivität gegen HSV 1 und 2, Influenza, HBV, VZV, EBV sowie retrovirale Infektionen.

PFA/PAA und ihre Derivate stellen unter Umständen eine effektive Alternative/Ergänzung zu Nucleosiden dar, da sie ein breites Spektrum von DNAund RNA-Polymerasen sowie die RT von Retroviren mit hinreichender Selektivität hemmen.

PFA und PAA selbst zeigen aufgrund ihrer Ähnlichkeit zu Pyrophosphat eine Toxizität durch Akkumulation im Knochen.

Die Verbindungen der vorliegenden Erfindung weisen ebenfalls wertvolle pharmakologische Eigenschaften auf. Insbesondere eignen sie sich zur Therapie und Prophylaxe von Infektionen, die durch DNA-Viren wie z.B. das Herpes-Simplex-Virus, das Human-Herpes-Virus 6, das Zytomegalie-Virus, Papova-Viren, das Varicella-Zoster-Virus, die Hepatitis-Viren oder Epstein-Barr-Virus, das Influenza-Virus oder RNA-Viren wie Toga-Viren oder insbesondere Retroviren wie die Onko-Viren HTLV-I und II, sowie die Lentiviren Visna und Humanes-Immunschwäche-Virus HIV-1 und 2, verursacht werden.

Besonders geeignet erscheinen die Verbindungen der Formel I zur Behandlung der klinischen Manifestationen der retroviralen HIV-Infektion beim Menschen, wie der anhaltenden generalisierten Lymphadenopathie (PGL), dem fortgeschrittenen Stadium des AIDS-verwandten Komplex (ARC) und dem klinischen Vollbild von AIDS, sowie von assoziierten CMV- und HSV-Infektionen.

Für Foscarnet (Phosphonoameisensäure-trinatriumsalz/PFA) ist in J. Infect. Dis. 172, 225 (1995) der antivirale/antiretrovirale Effekt in HIV-Patienten mit CMV-Retinitis beschrieben.

Der antivirale Effekt in murinem CMV ist in Antiviral Res 26, 1 (1995) beschrieben.

Weiterhin wird in JAMA 273, 1457 (1995) PFA zur Behandlung von CMV-Retinitis genutzt.

PFA- und PAA-2',3'-Didesoxy-3'-thiacytidin-Konjugate, die eine Inhibierung der HIV-1-Replikation zeigen, sind in J. Med. Chem. 37, 2216 (1994) dargestellt und in J Pharm. Sci. 83, 1269 (1994) sind Acyloxyalkylester von Foscarnet beschrieben.

Von besonderem Interesse sind jedoch die US-Anmeldung 5, 194, 654 bzw. die PCT-Anmeldung WO 94/13682. Hierin sind Lipid-Derivate von Phosphonocarbonsäuren beschrieben und deren Verwendung in Liposomen unter Bildung eines besonders stabilen liposomalen Komplexes. Neben einem äußerst breiten und sehr spekulativen Anspruch sind als Kern der Anmeldung 1-O-Alkylsn-glycero-3-phosphonocarbonsauren beschrieben, die besonders gut in die Lipiddoppelschicht von Liposomen eingebaut werden. Die beanspruchten Alkylreste können 2-24 Kohlenstoffatome umfassen.

Als Beispiel beschrieben und mit Daten für eine antivirale Wirkung belegt ist nur die Verbindung 1-O-Octadecyl-sn-glycero-3-phosphonoformiat (Batyl-Phosphonoformiat). Diese Verbindung hat sich bei den Untersuchungen und bei der Herstellung als nicht stabil herausgestellt. Im Gegensatz zu den genannten Patentanmeldungen werden die Verbindungen als Reinsubstanz in Lösung/Suspension verwendet, nicht in Liposomen.

In WO 96/15132 sind Lipidderivate von Phosphonoalkylcarbonsäuren beschrieben, die antiviral wirken sollen. Aus der Vielzahl von Verbindungen die von den dargestellten allgemeinen Formeln umfasst sind, sind beispielhaft als viral wirksam nur Verbindungen beschrieben, welche 1-O-Alkyl-propandiol-3-phosphono-formiat oder -acetat Struktur aufweisen, wobei die 2-Stellung des Propans noch eine OH, O-CH₃ oder O-Benzyl-Gruppe aufweisen kann. Verbindungen in denen eine 1-S-Alkylgruppe oder in 2-Stellung eine längerkettige O-Alkylgruppe vorkommt sind nur von der Formel umfasst aber nicht detailliert beschrieben.

Die erfindungsgemäßen Verbindungen der allgemeinen Formel I sind unter den gleichen Bedingungen stabil und zeigen sowohl in vitro als auch in vivo (MCMV-Modell in der Maus) eindeutige Vorteile.

Überraschenderweise wurde eine sehr enge Strukturwirkungsbeziehung bezüglich der Kettenlänge der verwendeten gesättigten Alkylreste festgestellt. Nur die Verwendung von zwei Alkylresten im Kettenlängenbereich von 10-13-Kohlenstoffatomen zeigen optimale Effekte.

Die beanspruchten Verbindungen dieser Anmeldung stellen daher eine nicht zu erwartende Verbesserung bezüglich WO 94/13682 und US 5, 194, 654 dar, wobei sie von diesen Anmeldungen zwar umfaßt sind, jedoch nicht den Kern der Anmeldung darstellen und auch nicht explizit genannt bzw. namentlich erwähnt sind oder deren Verwendung nahegelegt wäre.

Die Verbindungen der Formel I sind neu. Neben der besseren Stabilität (in Substanz und in Losung) der beanspruchten Verbindungen zeigen diese auch eine bessere Wirkung im Vergleich zu den bekannten Lipid-Derivaten.

Überraschenderweise besitzen die pharmazeutischen Wirkstoffe der Formel I im Vergleich zu den pharmakologisch aktiven freien bzw. unmodifizierten Substanzen eine größere therapeutische Breite. Sie verbessern darüber hinaus deren Verweilzeit im Körper, die Bioverfügbarkeit oder die oft als kritischer Faktor bekannte Membrangängigkeit (z.B. Blut-Hirn-Schranke, Zellmembran, etc.) der pharmakologisch aktiven Substanzen. Verbindungen der Formel I dienen somit als Trägersystem (Carrier) für die pharmakologisch aktiven Substanzen. Die Konjugate der Formel I können hinsichtlich ihrer Funktion als intrazelluläres Drug-Storage-, Drug-Targeting- und Drug-Delivery-System bezeichnet werden. Sie bewirken, daß die pharmakologisch aktive Substanz nach oraler Applikation intrazellulär freigesetzt wird, wobei diese Freisetzung in vorteilhafter Weise nicht unspezifisch in allen Zellen, Organen oder Geweben des Körpers stattfindet, sondern gezielt in solchen Zellen, die ein bestimmtes Enzym enthalten. Besonders überraschend ist jedoch, daß die Spaltung nicht bereits schon während des Transportes des Substrates durch die Körperflüssigkeiten, wie z.B. Blut, Serum oder Lymphflüssigkeit oder durch die Leber, erfolgt, sondern erst an oder in den entsprechenden Zielzellen. Auf diese Weise wird das unerwünschte Ausscheiden der Phosphonocarbonsäure durch die Niere oder Spaltung des Konjugats in der Leber vermieden, so daß der weitaus größte Teil des Wirkstoffes an bzw in die jeweiligen Zielzellen transportiert wird. Derartige Zellen sind, wie oben bereits erwähnt, insbesondere physiologisch oder pathophysiologisch aktivierte Zellen, die als Zielobjekt für die Verabreichung von pharmakologisch aktiven Substanzen in Frage kommen, wie beispielsweise Blutleukozyten, Lymphozyten, Makrophagen und andere Zellpopulationen des immunologisch lymphatischen Systems. Insbesondere handelt es sich hierbei um aktivierte Zellen (z.B. Makrophagen, Granulozyten, Lymphozyten, Leukozyten, Thrombozyten, Monozyten etc.), die beim jeweiligen Krankheitsgeschehen eine pathophysiologische oder symptomatische Rolle spielen. Daruber hinaus handelt es sich auch um Zellen, die durch Viren, Bakterien, Pilze oder andere Mikroorganismen infiziert sind.

Uberraschenderweise wurde auch gefunden, daß die therapeutische Breite einer pharmakologisch aktiven Phosphonocarbonsäure und deren Ester signifikant verbessert wird, wenn die Substanz an ein sehr spezielles lipidartiges Trägermolekül gekoppelt wird. Das so hergestellte Konjugat dient als neuer Wirkstoff für die Herstellung von pharmazeutischen Darreichungsformen. Insgesamt resultiert aus der Kopplung eine verstärkte Wirkung der pharmazeutisch aktiven Phosphonocarbonsäure in vivo, da durch das entstehende Drug-Delivery-Transport-System eine Lokalisierung der pharmakologische aktiven Substanz in Zielzellen erfolgt und dadurch die pharmakologisch aktive Substanz hinsichtlich ihrer Effizienz und Verträglichkeit verbessert wird. Dies bedeutet, daß einerseits die Menge der zu verabreichenden pharmakologisch aktiven Phosphonocarbonsäure reduziert werden kann oder andererseits unter Beibehaltung der gleichen effektiven Menge eine verstärkte pharmakologische Wirkung erzielt wird.

Aus dem Konjugat wird die pharmakologisch aktive Phosphonocarbonsäure durch enzymatische Hydrolyse des Konjugats freigesetzt.

Die Konjugate der Formel I zeigen entscheidende Vorteile im Vergleich zur nicht konjugierten pharmakologisch aktiven Phosphonocarbonsäure bzw. deren Ester. Der spezifische, kovalent an die pharmakologisch aktive Substanz gebundene Carrier verbessert die Bioverfügbarkeit der schlecht resorbierten pharmakotogisch aktiven Substanzen, die Vertraglichkeit der potentiell toxischen Wirkmolekülen, die Verweilzeit der schnell eliminierten oder metabolisierten Arzneimitteln und die Membranpenetration der schlecht membrangängigen Verbindungen (z.B. Blut-Hirn, Zellen etc.).

Die enzymatische Spaltung des Lipidteils in vivo erfolgt in der Regel nicht im Serum sondern erst intrazellulär Außerdem verbessert der Carrierteil mit seiner lecithinartigen Struktur, die für den beanspruchten Effekt essentiell ist, die Penetration oder Membrangängigkeit der pharmakologisch aktiven Substanz und zeigt einen Depoteffekt. Darüber hinaus ist die gastrointestinale Verträglichkeit der Lipidkonjugate vielfach besser als die der reinen pharmakologisch aktiven Phosphonocarbonsaure. Auch bei der Resorption zeigt das Lipid-Konjugat eine bessere Penetration durch Membranstrukturen und somit eine bessere Uberwindung der Resorptionsbarrieren. Entsprechendes gilt fur die Penetration z.B. der Blut-Hirn-Schranke

Durch eine bessere Bindung des Konjugats an Plasma- und Gewebeproteine wird ferner die Verteilung in vivo verbessert. Durch normale Biotransformation wird das Konjugat primär vom Thioether (n = 0) zum Sulfoxid (n = 1) oxidiert, was aber aufgrund der equipotenten Wirkung des Sulfoxids im Vergleich zum Thioether keinen Nachteil darstellt. Durch eine langsame Freisetzung der pharmakologisch aktiven Phosphonocarbonsäure aus dem Konjugat wird ein niedriger, aber über einen längeren Zeitraum konstanter aktiver Substanzspiegel gewährleistet und somit die Wirkung verbessert und/oder toxische Nebeneffekte vermieden. Die freigesetzte pharmakologisch aktive Substanz in Form des Monophosphats penetriert wegen seiner großen Hydrophilie nicht mehr aus der Zelle heraus

Sowohl die Gesamtkörper-, Zell- als auch die Organ-Halbwertszeiten der pharmakologisch aktiven Substanz werden durch Konjugation aufgrund der längeren Verweilzeit des Konjugats im Organismus erheblich verlängert. Aufgrund der fehlenden Spaltungsaktivität im Serum und in verschiedenen Organen ist nahezu keine bzw nur eine sehr geringere Knochenmark- und Organtoxizität zu beobachten Insbesondere ist vorteilhaft, daß die Konjugate der Formel I in verschiedenen Zielorganen, Geweben oder Zellen spezifisch angereichert werden

Die Verbindung der Formel I können als Wirkstoffe zur Herstellung von Arzneimitteln verwendet werden, die für alle Erkrankungen eingesetzt werden, bei denen hohe pharmakologisch aktive Substanzspiegel in Zellen, Organen oder Geweben erforderlich oder hilfreich sind Eine wesentliche Voraussetzung fur dieses als "Drug-Storage-Delivery-Targeting" zu bezeichnende System ist, daß die im Sinne der beabsichtigten Therapie anzusprechenden Zellen das Spaltungsenzym haben, so daß in einem ersten Schritt der Wirkstoff bindet und anschließend über die Zellmembran hinweg in das Innere der Zelle transportiert wird, wobei die Spaltung des Wirkstoffes zu der physiologisch aktiven Phosphonocarbonsäure entweder im wesentlichen gleichzeitig mit dem Transport über die Zellmembran oder auch spater teilweise innerhalb der Zelle erfolgt. Die intrazelluläre Spaltung kommt insbesondere in solchen Fällen vor, bei denen das Spaltungsenzym auch innerhalb der Zelle lokalisiert ist.

Geeignete Zielzellen sind beispielsweise Zellen des immunologisch-lymphatischen Systems (z.B. Blutleukozyten, Monozyten, Makrophagen, Lymphozyten) oder infizierte Zellen.

Überraschenderweise wurde auch gefunden, daß Verbindungen der allgemeinen Formel I die Vermehrung von DNA- bzw. RNA-Viren auf der Stufe der virusspezifischen DNA- bzw. RNA-Transkription hemmen. Die Substanzen können über die Inhibierung des Enzyms Reverse Transkriptase die Vermehrung von Retroviren beeinflussen (vgl. Proc. Natl. Acad. Sci. USA 83, 1911, 1986 bzw. Nature 325, 773, 1987). Von besonderem therapeutischen Interesse ist die Hemmwirkung auf das HI-Virus, dem Verursacher der Immunschwäche-Erkrankung AIDS. Zur Behandlung von AIDS ist heute u.a 3'-Azido-3'desoxythymidin (DE-A-3608606) bei AIDS Patienten zugelassen. Jedoch machen toxische Nebenwirkungen des 3'-Azido-3'-desoxythymidins auf das Knochenmark bei etwa 50 % der behandelten Patienten Bluttransfusionen erforderlich. Die Verbindungen der allgemeinen Formel I besitzen diese Nachteile nicht. Sie wirken antiviral, ohne in pharmakologisch relevanten Dosen zytotoxisch zu sein

Die Verbindungen der vorliegenden Erfindung und ihre pharmazeutischen Zubereitungen können auch in Kombination mit anderen Arzneimitteln zur Behandlung und Prophylaxe der oben genannten Infektionen eingesetzt werden. Beispiele dieser weiteren Arzneimittel beinhalten Mittel, die zur Behandlung und Prophylaxe von HIV-Infektionen oder diese Krankheit begleitende Erkrankungen einsetzbar sind wie 3'-Azido-3'-desoxythymidin, 2',3'-Didesoxynucleoside wie z B. 2',3'-Didesoxycytidin, 2',3'-Didesoxyadenosin und 2',3'-Didesoxyinosin, acyclische Nucleoside (z.B. Acyclovir), nicht-nukleosidische RT-Inhibitoren, Proteaseinhibitoren wie z.B. Invirase, Interferone wie z.B Interferon α, β, γ, Cytokine und Interleukine (z.B. Interleukin 16), Chemokine wie z.B. MIP1α, MIP1β, CCl, renale Ausscheidungs-Inhibitoren wie z.B. Probenicid, Nucleosid-Transport-Inhibitoren wie z.B. Dipyridamol, als auch Immunmodulatoren wie z.B. Interleukin II oder Stimulierungs-Faktoren wie z.B. Granulozyten-Makrophagen-Kolonie stimulierende Faktoren (GM-CSF), Granulozyten-Kolonie stimulierende Faktoren (G-CSF, Neutropoetin), Thrombopoetin und thrombopoetin-ähnliche Faktoren. Die Verbindungen der vorliegenden Erfindung und das andere Arzneimittel können jeweils einzeln, gleichzeitig gegebenenfalls in einer einzigen oder zwei getrennten Formulierungen oder zu unterschiedlichen Zeiten verabreicht werden, so daß ein synergistischer Effekt erreicht wird.

Als mögliche Salze der Verbindungen der allgemeinen Formel I kommen vor allem Alkali-, Erdalkali- und Ammoniumsalze der Carboxyl- und Phosphonatgruppe in Frage. Als Alkalisalze sind Lithium-, Natrium- und Kaliumsalze bevorzugt. Als Erdalkalisalze kommen insbesondere Magnesium- und Calciumsalze in Frage. Unter Ammoniumsalzen werden erfindungsgemäß Salze verstanden, die das Ammoniumion enthalten, das bis zu vierfach durch Alkylreste mit 1-4 Kohlenstoffatomen und/oder Aralkylreste, bevorzugt Benzylreste, substituiert sein kann. Die Substituenten können hierbei gleich oder verschieden sein.

Unter Carbonsäureestern der Phosphoncarbonsäurenlipid-Derivate werden pharmakologisch vertretbare Ester verstanden, solche sind vorzugsweise Ester mit einem Benzyl, Cholin, Ethanolamin, Carnitin, C₅-C₇-Cycloalkylrest oder mit einem geradkettigen oder verzweigten Alkylrest mit 1-6 Kohlenstoffatomen, insbesondere der Methyl, Ethyl, Propyl, Butyl, Pentyl, Hexyl, Isopropyl, i-Butyl, t-Butyl, Neopentyl- oder Thexylrest. Ganz besonders bevorzugt sind Methyl, Ethyl, Propyl, Butyl, t-Butyl und Benzyl.

Die Lipidphosphonocarbonsäureester sind in vitro gleich wirksam wie die entsprechenden freien Carbonsäuren. In vivo zeigen sie hingegen eindeutige Vorteile, insbesondere bei oraler Verabreichung.

Die Carbonsäureester der Verbindungen der Formel I zeigen eine im Sauren geringere Zersetzung durch Decarboxylierung und einer damit verbundene bessere Bioverfügbarkeit. Die zu verabreichende Dosis kann demnach gegenüber der entsprechenden freien Carbonsäure nochmals reduziert werden.
Weiterhin wird die Membrangängigkeit verbessert, z.B. bei der Überwindung der Blut-Hirn-Schranke und beim Passieren der Zellmembran in die Zielzelle. Da der Carbonsäureester in vivo durch Esterasen erst gespalten werden muß, wird die Halbwertszeit im Serum verlängert.

In der allgemeinen Formel I bedeutet R¹ vorzugsweise eine geradkettige C₁₀-C₁₂-Alkylgruppe R¹ stellt insbesondere eine Decyl-, Undecyl-, Dodecyl- oder Tridecylgruppe dar.

n ist vorzugsweise eine der Zahlen 0 oder 1.
R² bedeutet vorzugsweise eine geradkettige C₉-C₁₂-Alkylgruppe,
R² stellt insbesondere eine Decyl-, Undecyl- oder Dodecylgruppe dar.

Bevorzugte gekoppelte Phosphonsäuren bzw. deren Ester in den beanspruchten Konjugaten der allgemeinen Formel I sind folgende Säuren und deren Ester:
- Phosphonoameisensäure
- Phosphonoessigsäure
- Phosphonopropionsäure

Besondere bevorzugte Lipidteile sind n = 0 und die Kombination R₁ = Decyl / R₂ = Dodecyl, R₁ = Undecyl / R₂ = Undecyl oder R₁ = Dodecyl / R₂ = Decyl, und außerdem noch R₁ = Undecyl / R₂ = decyl, R₁ = Tridecyl / R₂ = decyl, R₁ = Dodecyl / R₂ = Undecyl.

Die Verbindungen der allgemeinen Formel I können dargestellt werden, in dem man
1. eine Verbindung der allgemeinen Formel II, 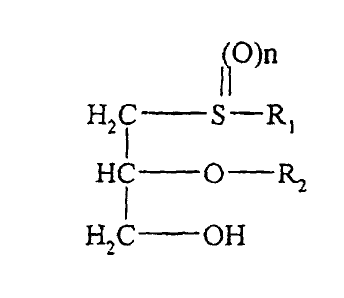 in der R¹, R² und n die angegebenen Bedeutungen besitzen, mit einer Verbindung der allgemeinen Formel III, 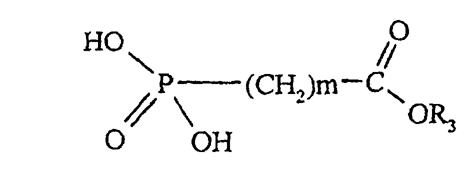 in der m die oben angegebene Bedeutung besitzt und R³ einen der oben angegebenen Esterreste darstellt, in Gegenwart von einem gegebenenfalls substituierten Arylsulfonsäurechlorid in einer organischen Base, bzw. in Gegenwart der Base in einem inerten organischen Lösungsmittel umsetzt und gegebenenfalls anschließend den Carbonsäureester durch alkalische Verseifung in ein Derivat der Formel I bzw. dessen physiologisch verträgliches Salz überführt; oder
2. ein gemischtes Anhydrid aus einer Verbindung der Formel III und einem Alkyl- oder Arylsulfonsäurechlorid herstellt und in Gegenwart einer Base in einem inerten organischen Lösungsmittel bzw. direkt in der Base mit einem Alkohol der Formel II zur Reaktion bringt und gegebenenfalls anschließend den Carbonsäureester alkalisch verseift; oder
3. eine Phosphonocarbonsäure der Formel III, in der R gleich Wasserstoff ist, mit einem Alkohol der Formel II in Gegenwart einer Base und eines gegebenenfalls substituierten Arylsulfonsäurechlorids umsetzt und nach Bedarf in ein physiologisch verträgliches Salz überführt; oder
4. ein gemischtes Anhydrid aus einer Verbindung der Formel III, in der R gleich Wasserstoff ist, und einem Alkyl- oder Arylsulfonsäurechlorid in Gegenwart einer Base, evtl. in einem inerten organischen Lösungsmittel, mit einem Alkohol der Formel II zur Reaktion bringt und das Konjugat gegebenenfalls in ein physiologisch verträgliches Salz überführt. oder
5. Phosphonsäuredichloride, der allgemeinen Formel IV, 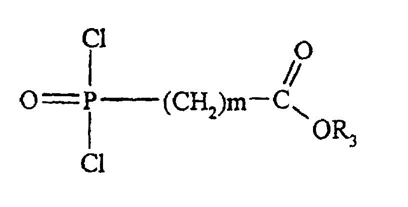 die sich nach Bhongle et al. (Synthetic Commun. **17**, 1071 (1987)) ausgehend von einem Phosphonsäure-bis-trimethylsilylester durch anschließende Umsetzung mit Oxalylchlorid darstellen lassen, mit einem Alkohol der allgemeinen Formel II in Gegenwart einer Base im Molverhältnis 1:1 umgesetzt.
   oder
6. eine Verbindung der Formel III mit Oxalylchlorid, wie in Tetrahedron Letters **33**, 7473(1992) beschrieben, in das entsprechende Phosphonsäuredichlorid der Formel IV überführt, das anschließend mit einem Alkohol der Formel II in Gegenwart einer Base im Molverhältnis 1:1 umgesetzt wird. Das als Zwischenprodukt entstehende Phosphonsäuremono-chlorid wird zum Halbester verseift und der Carbonsäureester durch alkalische Verseifung in ein Derivat der Formel I bzw. dessen physiologisch verträgliches Salz überführt.

Die freien Säuren der Lipidderivate von Phosphonocarbonsäuren können gegebenenfalls in die gewünschten Ester überführt werden.

Die Herstellung der Verbindungen der Formel II sind exemplarisch in den Beispielen und in EP-0545699 beschrieben.

Die Arzneimittel enthaltend Verbindungen der Formel I zur Behandlung von z.B. viralen Infektionen können in flüssiger oder fester Form enteral oder parenteral appliziert werden. Hierbei kommen die üblichen Applikationsformen in Frage, wie beispielsweise Tabletten, Kapseln, Dragees, Sirupe, Lösungen oder Suspensionen. Als Injektionsmedium kommt vorzugsweise Wasser zur Anwendung, das die bei Injektionslösungen üblichen Zusätze wie Stabilisierungsmittel, Lösungsvermittler und Puffer enthält. Derartige Zusätze sind z.B. Tartrat- und Zitratpuffer, Ethanol, Komplexbildner, wie Ethylen-diamintetraessigsäure und deren nicht toxischen Salze, hochmolekulare Polymere, wie flüssiges Polyethylenoxid zur Viskositätsregulierung. Flüssige Trägerstoffe für Injektionslösungen müssen steril sein und werden vorzugsweise in Ampullen abgefüllt. Feste Trägerstoffe sind beispielsweise Stärke, Lactose, Mannit, Methylcellulose, Talkum, hochdispere Kieselsäuren, höher molekulare Fettsäuren, wie Stearinsäure, Gelatine, Agar-Agar, Calziumphosphat, Magnesiumstearat, tierische und pflanzliche Fette, feste hochmolekulare Polymere, wie Polyethylenglykole, etc.. Für orale Applikationen geeignete Zubereitungen können gewünschtenfalls Geschmacks- und Süßstoffe enthalten.

Prinzipiell können die Verbindungen der Formel I oral, intratracheal, rektal, nasal, vaginal, lingual, i.v., intraarterial, i.m., intradermal oder s.c. verabreicht werden. Die Dosierung kann von verschiedenen Faktoren, wie Applikationsweise, Spezies, Alter oder individuellem Zustand abhängen. Die erfindungsgemäßen Verbindungen werden üblicherweise in Mengen von 0,1 - 1000 mg, vorzugsweise 2 - 800 mg besonders bevorzugt zwischen 30 mg und 250 mg pro Tag und pro kg Körpergewicht appliziert. Bevorzugt ist es, die Tagesdosis auf 2-5 Applikationen zu verteilen, wobei bei jeder Applikation 1-2 Tabletten mit einem Wirkstoffgehalt von 0,5 - 3000 mg verabreicht werden. Die Tabletten können auch retardiert sein, wodurch sich die Anzahl der Applikationen pro Tag auf 1-3 vermindert. Der Wirkstoffgehalt der retardierten Tabletten kann 20-5000 mg betragen. Der Wirkstoff kann auch durch Dauerinfusion gegeben werden, wobei die Mengen von 5-10000 mg pro Tag normalerweise ausreichen.

Im Sinne der vorliegenden Erfindung kommen außer den in den Beispielen genannten Verbindungen und der durch Kombination aller in den Ansprüchen genannten Bedeutungen der Substituenten die folgenden Verbindungen der Formel I in Frage:
1. (3-Dodecylmercapto-2-decyloxy) propoxy phosphinylhydroxyameisensäure
2. (3-Dodecylsulfinyl-2-decyloxy) propoxy phosphinylhydroxyameisensäure
3. (3-Dodecylsulfonyl-2-decyloxy) propoxy phosphinylhydroxyameisensäure
4. (3-Undecylmercapto-2-decyloxy) propoxy phosphinylhydroxyameisensäure
5. (3-Decylmercapto-2-decyloxy) propoxy phosphinylhydroxyameisensäure
6. (3-Tridecylmercapto-2-decyloxy) propoxy phosphinylhydroxyameisensäure
7. (3-Undecylmercapto-2-undecyloxy) propoxy phosphinylhydroxyameisensäure
8. (3-Undecylsulfinyl-2-undecyloxy) propoxy phosphinylhydroxyameisensäure
9. (3-Undecylsulfonyl-2-undecyloxy) propoxy phosphinylhydroxyameisensäure
10. (3-Dodecylmercapto-2-undecyloxy) propoxy phosphinylhydroxyameisensäure
11. (3-Decylmercapto-2-dodecyloxy) propoxy phosphinylhydroxyameisensäure
12. (3-Undecylmercapto-2-dodecyloxy) propoxy phosphinylhydroxyameisensäure
13. (3-Dodecylmercapto-2-dodecyloxy) propoxy phosphinylhydroxyameisensäure
14. (3-Dodecylmercapto-2-nonyloxy) propoxy phosphinylhydroxyameisensäure
15. (3-Undecylmercapto-2-nonyloxy) propoxy phosphinylhydroxyameisensäure
16. (3-Dodecylmercapto-2-octyloxy) propoxy phosphinylhydroxyameisensäure
17. (3-Dodecylmercapto-2-decyloxy) propoxy phosphinylhydroxypropionsäure
18. (3-Dodecylsulfinyl-2-decyloxy) propoxy phosphinylhydroxypropionsäure
19. (3-Dodecylsulfonyl-2-decyloxy) propoxy phosphinylhydroxypropionsäure
20. (3-Undecylmercapto-2-decyloxy) propoxy phosphinylhydroxypropionsäure
21. (3-Decylmercapto-2-decyloxy) propoxy phosphinylhydroxypropionsäure
22. (3-Tridecylmercapto-2-decyloxy) propoxy phosphinylhydroxypropionsäure
23. (3-Undecylmercapto-2-undecyloxy) propoxy phosphinylhydroxypropionsäure
24. (3-Undecylsulfinyl-2-undecyloxy) propoxy phosphinylhydroxypropionsäure
25. (3-Undecylsulfonyl-2-undecyloxy) propoxy phosphinylhydroxypropionsäure
26. (3-Dodecylmercapto-2-undecyloxy) propoxy phosphinylhydroxypropionsäure
27. (3-Decylmercapto-2-dodecyloxy) propoxy phosphinylhydroxypropionsäure
28. (3-Undecylmercapto-2-dodecyloxy) propoxy phosphinylhydroxypropionsäure
29. (3-Dodecylmercapto-2-dodecyloxy) propoxy phosphinylhydroxypropionsäure
30. (3-Dodecylmercapto-2-nonyloxy) propoxy phosphinylhydroxypropionsäure
31. (3-Undecylmercapto-2-nonyloxy) propoxy phosphinylhydroxypropionsäure
32. (3-Dodecylmercapto-2-octyloxy) propoxy phosphinylhydroxypropionsäure
33. (3-Dodecylmercapto-2-decyloxy) propoxy phosphinylhydroxyessigsäure
34. (3-Dodecylsulfinyl-2-decyloxy) propoxy phosphinylhydroxyessigsäure
35. (3-Dodecylsulfonyl-2-decyloxy) propoxy phosphinylhydroxyessigsäure
36. (3-Undecyimercapto-2-decyloxy) propoxy phosphinylhydroxyessigsäure
37. (3-Decylmercapto-2-decyloxy) propoxy phosphinylhydroxyessigsäure
38. (3-Tridecylmercapto-2-decyloxy) propoxy phosphinylhydroxyessigsäure
39. (3-Undecylmercapto-2-undecyloxy) propoxy phosphinylhydroxyessigsäure
40. (3-Undecylsulfinyl-2-undecyloxy) propoxy phosphinylhydroxyessigsäure
41. (3-Undecylsulfonyl-2-undecyloxy) propoxy phosphinylhydroxyessigsäure
42. (3-Dodecylmercapto-2-undecyloxy) propoxy phosphinylhydroxyessigsäure
43. (3-Decylmercapto-2-dodecyloxy) propoxy phosphinylhydroxyessigsäure
44. (3-Undecylmercapto-2-dodecyloxy) propoxy phosphinylhydroxyessigsäure
45. (3-Dodecylmercapto-2-dodecyloxy) propoxy phosphinylhydroxyessigsäure
46 (3-Dodecylmercapto-2-nonyloxy) propoxy phosphinylhydroxyessigsäure
47. (3-Undecylmercapto-2-nonyloxy) propoxy phosphinylhydroxyessigsäure
48. (3-Dodecylmercapto-2-octyloxy) propoxy phosphinylhydroxyessigsäure
49. (3-Dodecylmercapto-2-decyloxy) propoxy phosphinylhydroxyameisensäuremethylester
50. (3-Undecylmercapto-2-decyloxy) propoxy phosphinylhydroxyameisensäuremethylester
51. (3-Tridecylmercapto-2-decyloxy) propoxy phosphinylhydroxyameisensäuremethylester
52. (3-Undecylmercapto-2-undecyloxy) propoxy phosphinylhydroxyameisensäuremethylester
53. (3-Dodecylmercapto-2-undecyloxy) propoxy phosphinylhydroxyameisensäuremethylester
54. (3-Decylmercapto-2-dodecyloxy) propoxy phosphinylhydroxyameisensäuremethylester
55. (3-Dodecylmercapto-2-decyloxy) propoxy phosphinylhydroxyessigsäuremethylester
56. (3-Undecylmercapto-2-decyloxy) propoxy phosphinylhydroxyessigsäuremethylester
57. (3-Tridecylmercapto-2-decyloxy) propoxy phosphinylhydroxyessigsäuremethylester
58. (3-Undecylmercapto-2-undecyloxy) propoxy phosphinylhydroxyessigsäuremethylester
59. (3-Dodecylmercapto-2-undecyloxy) propoxy phosphinylhydroxyessigsäuremethylester
60. (3-Decylmercapto-2-dodecyloxy) propoxy phosphinylhydroxyessigsäuremethylester
61. (3-Dodecylmercapto-2-decyloxy) propoxy phosphinylhydroxyameisensäureethylester
62. (3-Undecylmercapto-2-decyloxy) propoxy phosphinylhydroxyameisensäureethylester
63. (3-Tridecylmercapto-2-decyloxy) propoxy phosphinylhydroxyameisensäureethylester
64. (3-Undecylmercapto-2-undecyloxy) propoxy phosphinylhydroxyameisensäureethylester
65. (3-Dodecylmercapto-2-undeeyloxy) propoxy phosphinylhydroxyameisensäureethylester
66. (3-Decylmercapto-2-dodecyloxy) propoxy phosphinylhydroxyameisensäureethylester
67. (3-Dodecylmercapto-2-decyloxy) propoxy phosphinylhydroxyessigsäureethylester
68. (3-Undecylmercapto-2-decyloxy) propoxy phosphinylhydroxyessigsäureethylester
69. (3-Tridecylmercapto-2-decyloxy) propoxy phosphinylhydroxyessigsäureethylester
70. (3-Undecylmercapto-2-undecyloxy)propoxy phosphinylhydroxyessigsäureethylester
71. (3-Dodecylmercapto-2-undecyloxy)propoxy phosphinylhydroxyessigsäureethylester
72. (3-Decylmercapto-2-dodecyloxy) propoxy phosphinylhydroxyessigsäureethylester
73. (3-Dodecylmercapto-2-decyloxy) propoxy phosphinylhydroxyameisensäureisopropylester
74. (3-Undecylmercapto-2-decyloxy) propoxy phosphinylhydroxyameisensäureisopropylester
75. (3-Tridecylmercapto-2-decyloxy) propoxy phosphinylhydroxyarneisensäureisopropylester
76. (3-Undecylmercapto-2-undecyloxy) propoxy phosphinylhydroxyameisensäureisopropylester
77. (3-Dodecylmercapto-2-undecyloxy) propoxy phosphinylhydroxyameisensäureisopropylester
78. (3-Decylmercapto-2-dodecyloxy) propoxy phosphinylhydroxyameisensäureisopropylester
79. (3-Dodecylmercapto-2-decyloxy) propoxy phosphinylhydroxyessigsäureisopropylester
80. (3-Undecylmercapto-2-decyloxy) propoxy phosphinylhydroxyessigsäureisopropylester
81. (3-Tridecylmercapto-2-decyloxy) propoxy phosphinylhydroxyessigsäureisopropylester
82. (3-Undecylmercapto-2-undecyloxy) propoxy phosphinylhydroxyessigsäureisopropylester
83. (3-Dodecylmercapto-2-undecyloxy) propoxy phosphinylhydroxyessigsäureisopropylester
84. (3-Decylmercapto-2-dodecyloxy) propoxy phosphinylhydroxyessigsäureisopropylester
85. (3-Dodecylmercapto-2-decyloxy) propoxy phosphinylhydroxyameisensäureneopentylester
86. (3-Undecylmercapto-2-decyloxy) propoxy phosphinylhydroxyameisensäureneopentylester
87. (3-Tridecylmercapto-2-decyloxy) propoxy phosphinylhydroxyameisensäureneopentylester
88. (3-Undecylmercapto-2-undecyloxy) propoxy phosphinylhydroxyameisensäureneopentylester
89. (3-Dodecylmercapto-2-undecyloxy) propoxy phosphinylhydroxyameisensäureneopentylester
90. (3-Decylmercapto-2-dodecyloxy) propoxy phosphinylhydroxyameisensäureneopentylester
91. (3-Dodecylmercapto-2-decyloxy) propoxy phosphinylhydroxyessigsäureneopentylester
92. (3-Undecylmercapto-2-decyloxy) propoxy phosphinylhydroxyessigsäureneopentylester
93. (3-Tridecylmercapto-2-decyloxy) propoxy phosphinylhydroxyessigsäureneopentylester
94. (3-Undecylmercapto-2-undecyloxy) propoxy phosphinythydroxyessigsäureneopentylester
95. (3-Dodecylmercapto-2-undecyloxy) propoxy phosphinylhydroxyessigsäureneopentylester
96. (3-Decylmercapto-2-dodecyloxy) propoxy phosphinylhydroxyessigsäureneopentylester
97. (3-Dodecylmercapto-2-decyloxy) propoxy phasphinylhydraxyameisensäurebenzylester
98. (3-Undecylmercapto-2-decyloxy) propoxy phosphinylhydroxyameisensäurebenzylester
99. (3-Tridecylmercapto-2-decyloxy) propoxy phosphinylhydroxyameisensäurebenzylester
100. (3-Undecylmercapto-2-undecyloxy) propoxy phosphinylhydroxyameisensäurebenzylester
101. (3-Dodecylmercapto-2-undecyloxy) propoxy phosphinylhydroxyameisensäurebenzylester
102. (3-Decylmercapto-2-dodecyloxy) propoxy phosphinylhydroxyameisensäurebenzylester
103. (3-Dodecylmercapto-2-decyloxy) propoxy phosphinylhydroxyessigsäurebenzylester
104. (3-Undecylmercapto-2-decyloxy) propoxy phosphinylhydroxyessigsäurebenzylester
105. (3-Tridecylmercapto-2-decyloxy) propoxy phosphinylhydroxyessigsäurebenzylester
106. (3-Undecylmercapto-2-undecyloxy) propoxy phosphinylhydroxyessigsäurebenzylester
107. (3-Dodecylmercapto-2-undecyloxy) propoxy phosphinylhydroxyessigsäurebenzylester
108. (3-Decylmercapto-2-dodecyloxy) propoxy phosphinylhydroxyessigsäurebenzylester

### Beispiel 1

### R,S-(3-Dodecylmercapto-2-decylory)-propoxy-phosphinylhydroxyameisensäure Di-Natriumsalz (DMDOP-PFA) und der Methylester DMDOP-PFA-OMe

18.2 ml Phosphonoameisensäure-trimethylester werden in 140 ml Dichlormethan gelöst und unter Rühren mit 72.5 ml Brom-trimethylsilan versetzt. Die Mischung wird 2 Stunden bei Raumtemperatur gerührt, eingedampft, der Rückstand zweimal in Methanol aufgenommen und die Lösung jeweils wieder eingedampft. Der Rückstand wird in 30 ml absolutem Pyridin aufgenommen und mit einer Lösung von 48.7 g R,S-(3-Dodecylmercapto-2-decyloxy)-propan-1-ol versetzt. Die Mischung wird zur Trockene eingedampft, der Rückstand unter Rühren mit 47.1 g 2,4,6, Tri-isopropylbenzolsulfochlorid und 150 ml abs. Pyridin versetzt. Die anfänglich dicke Suspension wird nach ca. 30 min dünner und wird 25 Stunden bei Raumtemperatur gerührt.

Der Niederschlag wird abgesaugt und mit etwas Pyridin gewaschen. Das Filtrat wird unter Rühren mit 150 ml Wasser versetzt, die Mischung 30 min bei Raumtemperatur gerührt, eingedampft und mit Ether versetzt. Der erneut ausgefallene Niederschlag wird abfiltriert und das Etherfiltrat mit 0.5 N HCl ausgeschüttelt. Die Etherphase wird gut mit Wasser gewaschen, getrocknet und eingedampft.

Der Rückstand (84.2 g) wird durch Chromatographie an Kieselgel mit Dichlormethan/Methanol/Eisessig (9:0.5:0.5) gereinigt. Die produkthaltigen Fraktionen werden eingedampft. Man erhält 45.4 g des entsprechenden R,S-(3-Dodecylmercapto-2-decyloxy)-propoxy-phosphinylhydroxyameisensäuremethylesters (DMDPO-PFA-OMe).

DC auf Kieselgel: R_{f}= 0.3 (Essigester/Aceton/Eisessig/Wasser 10:4:0.5:0.5)
R_{f}= 0.69 (Dichlormethan/Methanol 8:2)

Zur Verseifung des Carbonsäuremethylesters werden 5 g des oben erhaltenen Produktes in 70 ml Tetrahydrofuran gelöst und mit 6.7 ml 2 N NaOH versetzt. Man rührt 4 Stunden und läßt über Nacht stehen. Das Reaktionsgemisch wird mit 2-Ethylhexansäure auf pH 8 abgepuffert und eingedampft. Der Rückstand wird mit Aceton ausgerührt und das ausgefallene Produkt abgesaugt. Man erhält 4.1 g der Säure vom Fp. 242 - 246 C (Zersetzung).

### DC auf Kieselgel:

R_{f}= 0.31 (Isopropanol/Butylacetat/Wasser/konz. Ammoniak 10:6:3:1)
¹³C-NMR in D₂O: COOH (d,175 ppm, J_{P-C}= 231.4 Hz)

### Beispiel 2

### R,S-(3-Dodecylmercapto-2-decyloxy)-propoxy-phosphinylhydroxyessigsäure Di-Natriumsalz) (DMDOP-PAA) und der Methylester DMDOP-PAA-OMe.

Analog Beispiel 1 erhält man ausgehend von Phosphonoessigsäure-tri-methylester als wachsartiges Produkt und (3-Dodecylmercapto-2-decyloxy)-propan-1-ol die Titelverbindung vom Fp. 358 - 360 C (Zersetzung).

### DMDOP-PAA:

DC auf Kieselgel:
R_{f}= 0.53 (n-Butanol/Eisessig/Wasser 2:1:1)
R_{f}= 0.07 (Dichlormethan/Eisessig/Wasser 9:0.5:0.5)
DMDOP-PAA-OMe: DC auf Kieselgel
R_{f}= 0.6 (u-Butanol / Eisessig / Wasser 2:1:1)
R_{f}= 0.1 (Dichlormethan / Eisessig / Wasser 9:0,5:0,5)

### Beispiel 3

### Bestimmung der Knochenmarktoxizität in vitro (CFU-GM-Assay)

CFU-GM-Assays wurden, wie von Seidel und Kreja (Seidl, H. und J. Kreja, L., Blut **47**, 139 - 145, 1983) beschrieben, durchgeführt. Knochenmarkzellen (1 x 10⁵ Zellen/ml) von Balb/c-Mäusen wurden kultiviert in Iscove-Medium, das 0.8 % Methylzellulose, 20 % Pferdeserum, 10⁻⁴ M α-Thioglycerol und ein optimales Volumen (12.5 oder 25 µl) Endotoxin-aktivierten Mausserums, das von Balb/c-Mäusen 4 h nach I.v.-Injektion von 50 µg Endotoxin pro Tier (Salmonella abortus equi; Sigma, Deisenhofen, Deutschland) erhalten wurde, enthielt. Nach 6-tägiger Inkubation wurden die Kolonien mit 2-(p-iodophenyl)-3-(p-nitrophenyl)-5-phenyl Tetrazoliumchlorid-Hydrat (INT, Sigma) für weitere 24 h gefärbt und dann mit einem automatischen Image-Prozessor (Artek 982 B, Biosys GmbH, Karben, Deutschland) gezählt.

Tabelle 1 zeigt die IC₅₀-Konzentrationen aus mehreren konzentrationsabhängig durchgeführten Experimenten für Phosphonoameisensäure, DMDOP-PFA, Phosphonoessigsäure, DMDOP-PAA, (3-Octadecyloxy-2-hydroxy)-propoxyphosphinylhydroxyameisensäureethylester (OOHP-PFAE) und (3-Octadecyloxy-2-hydroxy)-propoxy-phosphinylhydroxyameisensäure (OOHP-PFA) i. Vgl. zu den Zytostatika Cisplatin (Cis-DDP) und Doxorubicin. Wie aus der Tabelle hervorgeht, zeigen DMDOP-PFA und DMDOP-PAA bis zur höchsten geprüften Konzentration von 100 µg/ml keinerlei Toxizität auf Knochenmarkstammzellen der granulozytären/monozytären Reihe. Während dies auch zutrifft für die Phosphonoameisensäure, sind die Phosphonoessigsäure sowie die Konjugate OOHP-PFAE und OOHP-PFA toxischer als DMDOP-PFA und DMDOP-PAA.

**Tab. 1**

| IC₅₀-Werte (µg/ml) für Cis-DDP, Doxorubicin, Phosphonoameisensäure (Foscamet), DMDOP-PFA, Phosphonoessigsäure, DMDOP-PAA, OOHP-PFAE und OOHP-PFA im CFU-GM-Assay. | |
|---|---|
| Substanz | IC₅₀ (µg/ml)^{a} |
| Cis-DDP (Cisplatin) | 0.45 ± 0.11 (5) |
| Doxorubicin | 0.046 ± 0.007 (4) |
| Phosphonoameisensäure (Foscarnet) | > 100 (6) |
| DMDOP-PFA | > 100 (6) |
| Phosphonoessigsäure | 62.88 (2) |
| DMDOP-PAA | > 100 (2) |
| OOHP-PFAE | 59.35 (3) |
| OOHP-PFA | 96.49 (3) |

| | |
|---|---|
| a Mittelwert ± SEM; n, Anzahl der Experimente, die konzentrationsabhängig in Doppel- oder Dreifachbestimmung durchgeführt wurden. | |

### Beispiel 4

Bestimmung der oralen Bioverfügbarkeit in Nurinen Cytomegaly Virus (MCMV) Modell.

Weibliche imunokompromierte Balb/e Mäuse wurden mit einer Dosis i.p. von 8x10⁵ PFU (plaque forming units) behandelt. Die Überlebensrate der Tiere stieg in der Reihe unbehandelt < Foscarnet behandelt < DMDOP-PFA < DMDOP-PFA-OMe.

## Patentansprüche

1. Neue Phospholipid-Derivate von Phosphonocarbonsäuren der allgemeinen Formel I, 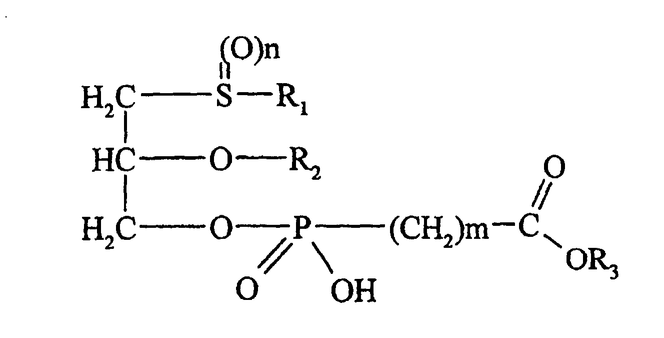 in der
R¹ eine geradkettige oder verzweigte, gesättigte oder ungesättigte Alkylkette mit 9-13 Kohlenstoffatomen,
R² eine geradkettige oder verzweigte, gesättigte oder ungesättigte Alkylkette mit 8-12 Kohlenstoffatomen sein kann,
R³ H, eine geradkettige oder verzweigte Alkykette mit 1-6 Kohlenstoffatomen,
im besondern Methyl, Ethyl, Propyl, Isopropyl, Butyl, Isobutyl, t-Butyl, Pentyl, Hexyl, Neopentyl, Thexyl oder Phenyl, Cholin, Ethanolamin, Carnitin, C₅-C₇-Cycloalkylrest, Benzyl oder eine der folgenden Gruppen 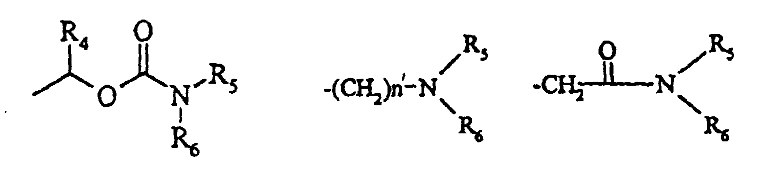 wobei R₄ Alkyl, Benzyl oder Phenyl und R₅ und R₆ Alkyl und n' 1,2 oder 3 bedeuten kann,
n 0, 1 oder 2 bedeutet und
m 0 bzw. für 1 bis 3 steht,
deren Tautomere, optischen Isomere, Racemate, deren Prodrugs und deren physiologisch verträgliche Salze anorganischer und organischer Basen.

2. Verbindungen der Formel I nach Anspruch 1, in denen R₁ eine Decyl-, Undecyl-, Dodecyl- oder Tridecylgruppe darstellt.

3. Verbindungen der Formel I nach einem der Ansprüche 1 oder 2, in dem R₂ eine Decyl-, Undecyl- oder Dodecylgruppe darstellt.

4. Verbindungen der Formel I nach einem der Ansprüche 1-3, in denen n 0 oder 1 darstellt.

5. Verbindungen der Formel I nach einem der Ansprüche 1-4, in denen m 0, 1 oder 2 darstellt.

6. Verbindungen der Formel I nach einem der Ansprüche 1-5, in denen R3 einen Methyl-, Ethyl-, Propyl-, Butyl-, t-Butyl- oder Benzylrest darstellt.

7. Arzneimittel enthaltend mindestens eine Verbindung der allgemeinen Formel 1 nach einem der Ansprüche 1-6 neben üblichen pharmazeutischen Hilfs- und Trägerstoffen.

8. Verwendung mindestens einer Verbindung der allgemeine Formel 1 nach einem der Ansprüche 1-6 zur Herstellung eines Medikaments zur Behandlung von Autoimmunkrankheiten, Neoplasmien, entzündlicher, viraler oder retroviraler Erkrankungen.

9. Verbindung gemäß Anspruch 1, in der
R₁ Dodecyl-, R₂ Decyl-, R₃ H oder Methyl,
n 0, m 0 oder 1 sind,
sowie ihre optischen Isomeren, Racemate und/oder Natriumsalze.

## Claims

1. New phospholipid derivatives of phosphono-carboxylic acids of the general formula I, 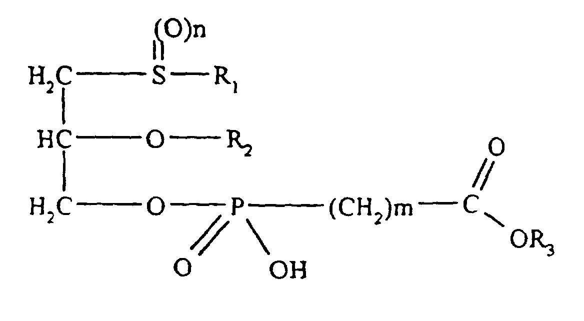 in which
R¹ is a straight-chained or branched, saturated or unsaturated alkyl chain with 9-13 carbon atoms
R² can be a straight-chained or branched, saturated or unsaturated alkyl chain with 8-12 carbon atoms
R³ represents H or a straight-chained or branched alkyl chain with 1-6 carbon atoms, preferrably methyl, ethyl, propyl, isopropyl, butyl, isobutyl, t-butyl, pentyl, hexyl, neopentyl, thexyl or phenyl, choline, ethanolamine, carnitine, C₅-C₇-cycloalkyl residue, benzyl or one of the following groups 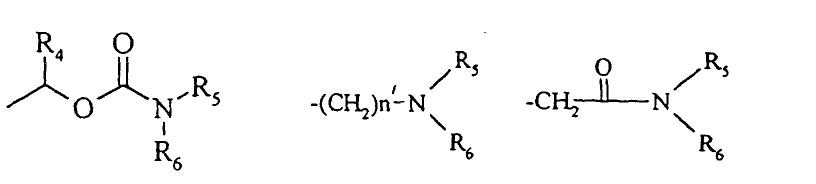
wherein
R₄ is alkyl, benzyl or phenyl and
R₅ and R₆ are alkyl and
n' is 1, 2 or 3
n denotes 0, 1 or 2 and
m represents 0 or 1 to 3,
their tautomers, optical isomers and racemates, prodrugs thereof and their physiologically tolerated salts of inorganic or organic bases.

2. Compound of formula I according to claim 1 in which R₁ represents a decyl, undecyl, dodecyl or tridecyl group.

3. Compound of formula I according to one of the claims 1 or 2 in which R² represents a decyl, undecyl or dodecyl group.

4. Compound of formula I according to one of the claims 1 to 3 in which n represents the number 0 or 1.

5. Compound of formula I according to one of the claims 1 to 4 in which m represents the number 0, 1 or 2.

6. Compound of formula I according to one of the claims 1 to 5 in which R₃ represents a methyl, ethyl, propyl, butyl, t-butyl, or benzyl group.

7. Pharmaceutical compostion containing at least one compound of the general formula I as claimed in one of the claims 1 to 6 in addition to the usual pharmaceutical auxiliary substances and carriers.

8. Use of at least one compound of the general formula I as claimed in one of the claims 1 to 6 for the production of a medicament for the treatment of autoimmune diseases, neoplasias, inflammatory, viral or retroviral diseases.

9. Compound according to claim 1 in which
R₁ is dodecyl
R₂ is decyl
R₃ is hydrogen or methyl
n is 0
m is 0 or 1
as well as its optical isomers, racemates and/or sodium salts thereof.

## Revendications

1. Nouveaux dérivés phospholipidiques d'acides phosphonocarboxyliques de formule générale I 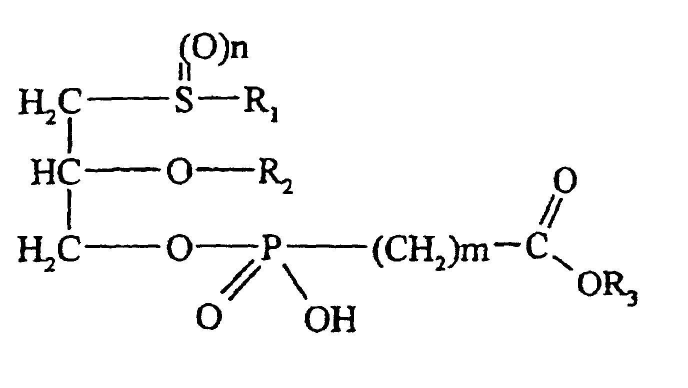 dans laquelle
R₁ représente une chaîne alkylique linéaire ou ramifiée, saturée ou insaturée, comportant de 9 à 13 atomes de carbone,
R₂ représente une chaîne alkylique linéaire ou ramifiée, saturée ou insaturée, comportant de 8 à 12 atomes de carbone,
R₃ représente H ou une chaîne alkylique linéaire ou ramifiée comportant de 1 à 6 atomes de carbone,
en particulier, les groupes méthyle, éthyle, propyle, isopropyle, butyle, isobutyle, t-butyle, pentyle, hexyle, néopentyle, thexyle ou phényle, choline, éthanolamine, carnitine, cycloalkyle en C₅-C₇, benzyle ou un des groupes suivants 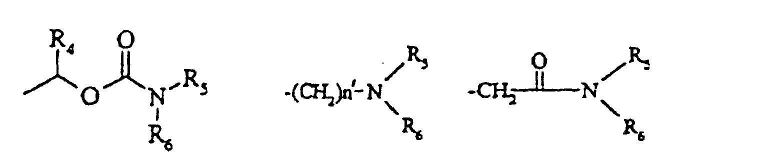 dans lesquels R₄ représente un groupe alkyle, benzyle ou phényle et R₅ et R₆ représentent un groupe alkyle et n'vaut 1, 2 ou 3,
n vaut 0, 1 ou 2, et
m vaut 0 ou présente une valeur de 1 à 3,
leurs tautomères, isomères optiques, racémates, précurseurs de médicaments et sels physiologiquement acceptables, formés avec des bases inorganiques ou organiques.

2. Composés de formule I selon la revendication 1, dans laquelle R₂ représente un groupe décyle, undécyle, dodécyle ou tridécyle.

3. Composés de formule I selon l'une des revendications 1 ou 2, dans laquelle R₂ représente un groupe décyle, undécyle ou dodécyle.

4. Composés de formule I selon l'une des revendications 1 à 3, dans laquelle n vaut 0 ou 1.

5. Composés de formule I selon l'une des revendications 1 à 4, dans laquelle m vaut 0, 1 ou 2.

6. Composés de formule. I selon l'une des revendications 1 à 5, dans laquelle R₃ représente un groupe méthyle, éthyle, propyle, butyle, t-butyle ou benzyle.

7. Médicament contenant au moins un composé de formule générale I selon l'une des revendications 1 à 6, en plus d'adjuvants et véhicules pharmaceutiques usuels.

8. Utilisation d'au moins un composé de formule générale I selon l'une des revendications 1 à 6, pour la préparation d'un médicament destiné au traitement de maladies autoimmunes, néoplasiques, inflammatoires, virales ou rétrovirales.

9. Composé selon la revendication 1, dans lequel
R₁ représente un groupe dodécyle,
R₂ représente un groupe décyle,
R₃ représente H ou un groupe méthyle,
n vaut 0,
m vaut 0 ou 1,
ainsi que ses isomères optiques, racémates et/ou sels de sodium.
